# FASCICULE DE BREVET EUROPEEN

(11) **EP 0 847 801 B1**
(45) Date de publication et mention de la délivrance du brevet: **04.07.2012**
(21) Numéro de dépôt: 97402930.8
(22) Date de dépôt: 04.12.1997
(51) Int. Cl.: B01J 23/86, B01J 23/26, C07C 17/20, C07C 19/08, B01J 23/94

(54) **Catalyseurs massiques à base de chrome et de nickel pour la fluoration en phase gazeuse d'hydrocarbures halogènes**
Vollkatalysatoren auch Chrom und Nickeloxyden für die Fluorierung von halogenierten Kohlenwasserstoffen
Chromium and nickel oxide-based solid catalysts useful for the fluorination of halogenated hydrocarbons

(30) Priorité: 13.12.1996 FR 9615360
(43) Date de publication de la demande: 17.06.1998
(73) Titulaire: ARKEMA FRANCE, 92700 Colombes (FR)
(72) Inventeur: Lacroix, Eric, 69480 Ambérieux d'Azergues (FR); Schirmann, Jean-Pierre, 75011 Paris (FR)
(74) Mandataire: Dang, Doris

(56) Documents cités:
- EP-A- 0 514 932
- EP-A- 0 546 883
- EP-A- 0 657 408
- WO-A-93/25507
- GB-A- 1 055 346

## Description

La présente invention concerne le domaine de la fluoration d'hydrocarbures halogénés et a pour objet le procédé de fluoration du perchloroéthylène en présence de catalyseurs massiques à base de chrome et de nickel.

Une des différentes voies d'accès aux hydrogénoalcanes -substituts des CFC (ChloroFiuoroCarbures)- est la fluoration en phase gazeuse avec HF. Pour cela, de nombreux catalyseurs sont décrits dans la littérature et bon nombre d'entre eux sont à base de chrome. Le passage des CFC aux substituts a induit une recherche sur les catalyseurs afin d'améliorer leurs performances aussi bien du point de vue activité que sélectivité.

Tout d'abord, des travaux ont été engagés afin d'améliorer les performances de catalyseurs à base de chrome. Ainsi, la demande de brevet EP 514 932 revendique comme catalyseur de fluoration un oxyde de chrome de grande surface qui, selon les auteurs, présente une grande activité et une longue durée de vie.

Parallèlement, des actions ont été engagées afin de modifier les performances de catalyseurs à base de chrome par ajout de dopants ou de cocatalyseurs. Ainsi, s'agissant des catalyseurs mixtes Ni-Cr, le brevet FR 2 669 022 revendique la synthèse du F134a (1,1,1,2-tétrafluoroéthane) par fluoration en phase gazeuse du F133a (1-chloro-2,2,2-trifluoroéthane) sur un catalyseur à base de dérivés du nickel et du chrome, supportés sur des alumines plus ou moins fluorées, voire sur du fluorure d'aluminium. La présence du support apporte au catalyseur certaines caractéristiques, notamment une certaine solidité. Par contre, les faibles quantités de matières actives risquent de limiter l'activité catalytique, voire la durée de vie du catalyseur; en outre, les faibles teneurs en métaux non précieux ne facilitent pas une récupération rentable des catalyseurs usagés.

Le brevet EP 546 883 décrit la préparation de catalyseurs massiques à base de chrome et de nickel par la méthode sol-gel en plusieurs étapes, la première consistant à former un sol mixte d'hydroxydes de chrome III et de nickel II. Cette technique partant d'un mélange des précurseurs du chrome et du nickel est relativement longue et coûteuse à mettre en oeuvre.

Dans la demande de brevet WO 93/25507, la préparation de catalyseurs à base de chrome et d'au moins un dérivé d'un métal de transition choisi parmi le nickel, le platine et le palladium est réalisée de diverses façons: imprégnation d'un support, coprécipitation, imprégnation d'un dérivé du chrome... Aucune caractéristique du catalyseur ou du support n'est fournie dans ce document.

Il a maintenant été trouvé qu'un catalyseur mixte Ni-Cr, particulièrement efficace pour la fluoration par HF en phase gazeuse d'hydrocarbures halogénés, saturés ou oléfiniques, peut être obtenu par simple imprégnation d'un oxyde de chrome III amorphe massique de grande surface et de volume poreux important, avec une solution d'un dérivé du nickel.

L'invention a donc pour objet des catalyseurs massiques à base de chrome et de nickel, obtenus par imprégnation d'un oxyde de chrome III amorphe avec une solution d'un dérivé du nickel, caractérisé en ce que l'oxyde de chrome massique utilisé présente une surface BET supérieure à 150 m²/g, de préférence supérieure à 180 m²/g, et un volume poreux (défini comme le volume des pores de rayon inférieur à 7,5 µm) supérieur à 0,15 ml/g, de préférence supérieur à 0,18 ml/g.

Un oxyde de chrome III de surface BET supérieure à 150 m²/g peut être synthétisé par les différentes techniques connues de l'homme de l'art. A titre non limitatif, on peut citer la calcination d'un précipité d'hydroxyde de chrome III, la formation d'un gel d'hydroxyde de chrome III suivie de sa calcination, la réduction du chrome VI par un alcool ou un autre réducteur, la décomposition thermique d'un dérivé oxydé du chrome tel que Cr03 et (NH4)2Cr207. On préfère utiliser un oxyde de chrome obtenu par calcination d'un hydroxyde de chrome III ou par réduction de l'oxyde de chrome VI. Des oxydes de chrome III du commerce peuvent convenir, sous réserve qu'ils présentent une surface et une porosité adéquates.

L'oxyde de chrome III peut se présenter sous différents formes (pastilles, extrudés, billes, ...). La forme retenue impose bien évidemment la forme du catalyseur final ; elle devra donc être inaltérée par l'étape d'imprégnation. Pour cela différents additifs (graphite, Cr203 cristallisé, ...) peuvent être ajoutés lors de la mise en forme, afin d'améliorer la solidité des particules de chrome.

L'imprégnation de l'oxyde de chrome III est réalisée au moyen d'une solution aqueuse ou alcoolique d'un précurseur du nickel qui peut être un oxyde, hydroxyde, halogénure, oxyhalogénure, nitrate, sulfate ou autre composé du nickel II, soluble en milieu aqueux ou alcoolique. Le composé préféré est le chlorure de nickel.

Le rapport atomique Ni/Cr dans le catalyseur final peut varier entre 0,01 et 1, de préférence entre 0,02 et 0,6. Un rapport atomique compris entre 0,02 et 0,4 est particulièrement avantageux.

L'imprégnation de l'oxyde de chrome peut être réalisée avant la mise en forme du catalyseur (imprégnation de la poudre de Cr203) ou sur l'oxyde de chrome III déjà mis en forme (billes, pastilles, extrudés, ...). Cette dernière technique est préférée lorsque la forme du catalyseur n'est pas altérée par l'étape d'imprégnation. L'imprégnation peut être réalisée selon les différentes techniques connues par l'homme de l'art (immersion, imprégnation avec un volume ajusté à la porosité du catalyseur, ...). L'imprégnation ajustée au volume poreux du catalyseur est la technique préférée. La solution d'imprégnation peut être une solution aqueuse ou une solution alcoolique. Lorsqu'il n'y a pas de problème de solubilité, la solution aqueuse est préférée ; on évite ainsi l'exothermie due à la réduction par l'alcool du chrome **VI** superficiel (toujours présent à faible teneur dans Cr₂O₃).

Afin d'optimiser l'activité du catalyseur, il convient de le soumettre à un prétraitement par HF en l'absence de composés organiques. L'oxyde de chrome **III** et les dérivés du nickel se fluorant en présence d'HF, il est nécessaire de réaliser cette fluoration en contrôlant l'exothermie de la réaction, afin d'éviter une dégradation du catalyseur (cristallisation, dégradation des billes, pastilles ou extrudés, ...). Un prétraitement (ou activation) type du catalyseur comporte d'abord une étape de séchage sous inerte (azote, hélium, ...) ou air à une température comprise entre 100 et 350°C, puis une étape d'activation par HF. Pour contrôler l'exothermie, l'HF est, d'une part, introduit à basse température (150-200°C) et, d'autre part, il est dilué dans de l'air ou, de préférence, dans un inerte. Après passage des "vagues d'exothermicité" dues à l'adsorption de l'HF sur le catalyseur, la température est progressivement augmentée pour atteindre 350-380°C et observer un palier à cette température. Lorsque la solidité du catalyseur le permet, celui-ci peut être activé en lit remué ou fluidisé; le contrôle de l'exothermie est ainsi plus aisé. Pour éviter tout dégradation du catalyseur, il est recommandé de ne pas dépasser une température de 400°C.

La température de fluoration est généralement comprise entre 50 et 500°C, mais souvent on opère de préférence à une température comprise entre 100 et 450°C et, plus particulièrement, entre 120 et 400°C.

Le temps de contact généralement est compris entre 3 et 100 secondes. Bien souvent, un bon compromis entre un taux de conversion et une productivité élevés impose un temps de contact inférieur à 30 secondes.

Le rapport molaire : HF/perchloroéthylène peut varier entre 1/1 et 30/1. Cependant, afin d'obtenir des productivités élevées, il est avantageusement inférieur à 20.

La pression de travail n'est pas critique, mais est en général comprise entre 0,08 et 2 MPa absolus et, de préférence, entre 0,1 et 1,5 MPa absolus.

Les catalyseurs peuvent fonctionner en lit fixe, mais également, lorsqu'ils le permettent, en lit fluide ou remué.

Lorsque la réaction de fluoration conduit à un encrassage du catalyseur (formation de "coke"), il est possible de réaliser la fluoration en injectant en continu un oxydant (air, oxygène,...). Lorsque le catalyseur est désactivé par cokage, il est également possible de le régénérer par un traitement à l'air, à l'oxygène ou par un mélange CI2/HF, à une température comprise entre 250 et 400°C.

Les exemples suivants illustrent l'invention.

### PREPARATION DES CATALYSEURS

### Exemple 1 : Catalyseur A

100 ml (139 g) d'un oxyde de chrome III commercial sous forme de pastilles présentant les caractéristiques suivantes :
- Surface BET {m²/g) : 223
- Volume poreux {r<7,5/ µm) : 0,272 ml/g
- graphite (liant de pastillage) : 4,1% poids
est imprégné à température ambiante et sous pression atmosphérique avec une solution de chlorure de nickel composée de 22,6 g de NiCl2.6H20 et 20 ml d'eau.

En fin d'imprégnation, la totalité de la solution est absorbée par le catalyseur. Celui-ci est ensuite séché à température ambiante, sous pression atmosphérique.

Ce catalyseur imprégné est alors séché sous azote à 200°C pendant 18 heures, puis une partie (70 ml) est activée par un mélange azote/HF en contrôlant la température afin de ne pas dépasser une exothermie de 30°C par rapport à la température de consigne. Progressivement le mélange est enrichi en HF et la température augmentée pour atteindre 380°C sous HF pur (1 mole/h d'HF). Finalement, le catalyseur est prétraité par HF pur dans ces conditions opératoires pendant 18 heures.

Le catalyseur ainsi imprégné, séché et activé, contient 3,5 % massique de nickel.

### Exemple 2 : Catalyseur B

Dans une solution aqueuse de chlorure de nickel préparée par dissolution de 75 g de NiCl₂.6H₂O et 35 ml d'eau, on immerge, à température ambiante 100 ml de l'oxyde de chrome **III** décrit à l'exemple 1. Le catalyseur est ensuite séché, puis activé selon la procédure de l'exemple 1.

Le catalyseur ainsi imprégné, séché et activé, contient 4,2 % massique de nickel.

### Exemple 3 : Catalyseur C

Ce catalyseur est préparé, séché et activé selon la procédure de l'exemple 1, sauf que la solution d'imprégnation est composée de 34 g de NiCl₂.6H₂O et 18 ml d'eau.

Après séchage et activation, le catalyseur ainsi imprégné contient 5,1 % massique de nickel.

### Exemple 4 comparatif : Catalyseur D sans nickel

L'oxyde de chrome III décrit dans l'exemple 1 est utilisé directement sans subir d'imprégnation au nickel. Avant le test de fluoration, le catalyseur est soumis à un prétraitement N₂/HF comparable à celui décrit dans l'exemple 1.

### Exemple 5 comparatif : Catalyseur E préparé à partir d'un oxyde de chrome ne respectant pas les critères de surface et porosité.

100 ml d'un oxyde de chrome commercialisé sous forme de poudre et présentant les caractéristiques suivantes :

| | | | |
|---|---|---|---|
| • | Surface BET (m²/g) | : | 66 |
| • | Volume poreux (r<7,5 µm) | : | 0,14 ml/g |

sont imprégnés par immersion dans une solution aqueuse de chlorure de nickel constituée par 75 g de NiCl₂.6H₂O et 35 ml d'eau. Le catalyseur est ensuite séché, puis activé selon la procédure de l'exemple 1.

Après séchage et activation, le catalyseur ainsi imprégné contient 3,6 % massiques de nickel.

### EXEMPLES DE FLUORATION

Les catalyseurs décrits dans les exemples 1 à 5 ont été utilisés pour la fluoration en phase gazeuse du perchloroéthylène (exemples 6 à 11) .

Les conditions opératoires et les résultats obtenus sont rassemblés dans le tableau suivant où les abréviations ont les significations suivantes :

| | | |
|---|---|---|
| F114 + F114a | : | dichlorotétrafluoroéthanes |
| F115 | : | chloropentafluoroéthane |
| F122 | : | 1,1-difluoro-1,2,2-trichloroéthane |
| F123 | : | 1,1-dichloro-2,2,2-trifluoroéthane |
| F123a | : | 1,2-dichloro-1,1,2-trifluoroéthane |
| F124 | : | 1-chloro-1,2,2,2-tétrafluoroéthane |
| F124a | : | 1-chloro-1,1,2,2-tétrafluoroéthane |
| F125 | : | pentafluoroéthane |
| F1111 | : | fluorotrichloroéthylène |

Les exemples 6 à 9 réalisés avec les catalyseurs A, B et C selon l'invention montrent que ces catalyseurs faciles à préparer (simple imprégnation d'un oxyde de chrome commercial) sont de très bons catalyseurs de fluoration ; de plus, la récupération de ces catalyseurs usagés est rentable compte tenu des teneurs en chrome.

Les essais de l'exemple 10 réalisés avec le catalyseur D traduisent, par comparaison avec les résultats obtenus avec les catalyseurs A, B et C, l'effet bénéfique du nickel.

Enfin, les essais de l'exemple 11 réalisés avec le catalyseur E montrent qu'un catalyseur préparé avec un oxyde de chrome qui ne respecte pas les critères de surface et volume poreux définis dans la présente invention donne des résultats de fluoration nettement moins performants.

**TABLEAU I**

| **Fluoration du perchloroéthylène** | | | | | | | | | | | |
|---|---|---|---|---|---|---|---|---|---|---|---|
| **EXEMPLE** | | **6** | | **7** | | **8** | **9** | **10 comparatif** | | **11 comparatif** | |
| **CONDITIONS OPERATOIRES** | | | | | | | | | | | |
| - Catalyseur | | A | A | A | A | B | C | D | D | E | E |
| - Température (°C) | | 300 | 350 | 280 | 280 | 280 | 300 | 350 | 300 | 300 | 350 |
| - Rapport molaire : HF/C₂Cl₄ | | 7,1 | 7,1 | 7,2 | 6,8 | 6,9 | 6,9 | 6,1 | 7,0 | 7,3 | 7,1 |
| - Rapport molaire : Oxygène/C₂Cl₄ | | 0,01 | 0,01 | 0,01 | 0,01 | 0,01 | 0,01 | 0,01 | 0,01 | 0,01 | 0,01 |
| - Pression (MPa) | | 0,1 | 0,1 | 0,1 | 0,1 | 0,1 | 0,1 | 0,1 | 0,1 | 0,1 | 0,1 |
| - Temps de contact (secondes) | | 5,2 | 4,9 | 5,4 | 5,4 | 5,5 | 5,3 | 5,5 | 5,1 1 | 5,3 | 5,4 |
| - Age du catalyseur (heures) | | 24 | 48 | 48 | 448 | 44 | 24 | 24 | 48 | 24 | 48 |
| **RESULTATS** | | | | | | | | | | | |
| - Taux de transformation global de C₂Cl₄ (%) | | 72,3 | 82,6 | 66,7 | 62,6 | 65,2 | 69,3 | 91,2 | 72,8 | 53,8 | 60,6 |
| - Sélectivité (% molaire) en : | | | | | | | | | | | |
| | F125 | 22,2 | 49,8 | 10,3 | 8,9 | 9,1 | 19,8 | 25,0 | 15,3 | 4,3 | 7,4 |
| | F124 | 32,3 | 16,7 | 31,3 | 34,6 | 29,0 | 32,5 | 11,6 | 32,3 | 23,1 | 30,6 |
| | F124a | 1,5 | 0,7 | 1,4 | 1,9 | 1,5 | 1,5 | 0,2 | 1,5 | 1,4 | 1,7 |
| | F123 | 30,5 | 13,5 | 42,0 | 37,7 | 44,5 | 32,3 | 22,0 | 36,1 | 47,2 | 37,4 |
| | F123a | 1,9 | 0,5 | 2,8 | 2,9 | 2,4 | 1,9 | 0,1 | 1,7 | 4,6 | 1,9 |
| | F122 | 3 | 0,7 | 5,4 | 5,5 | 6,2 | 3,6 | 0,2 | 2,5 | 10 | 5,6 |
| | F133a | 0,6 | 4,8 | 0,4 | 0,2 | 0,3 | 0,6 | 11,1 | 2,5 | 0,4 | 3,5 |
| | F115 | 0.5 | 5,3 | 0,2 | 0,1 | 0,1 | 0,7 | 21,3 | 2,1 | 0,5 | 4,8 |
| | F114 + F114a | 2,2 | 3,1 | 1,8 | 1,2 | 1,6 | 2,1 | 7,0 | 3,0 | 1,6 | 3,1 |
| | F1111 | 4,6 | 4,1 | 4,2 | 6,5 | 5,0 | 4,9 | 0,9 | 2,9 | 6,5 | 3,1 |
| | Autres | 0,7 | 0,8 | 0,2 | 0,5 | 0,3 | 0,1 | 0,6 | 0,1 | 0,4 | 0,9 |

## Revendications

1. Procédé de fluoration catalytique de perchloroéthylène par HF en phase gazeuse, comprenant : la préparation d'un catalyseur massique à base de chrome et de nickel comprenant l'imprégnation d'un oxyde de chrome III amorphe avec une solution d'un dérivé du nickel, **caractérisé en ce que** l'oxyde de chrome utilisé présente une surface BET supérieure à 150 m²/g et un volume poreux défini comme le volume des pores de rayon inférieur à 7,5µm supérieur à 0,15 ml/get **en ce que** le rapport atomique Ni/Cr est compris entre 0,02 et 0,4 et l'utilisation de ce catalyseur pour la dite fluoration.

2. Procédé selon la revendication 1 **caractérisé en ce que** l'oxyde de chrome a une surface BET supérieure à 180 m²/g.

3. Procédé selon la revendication 1 ou 2 **caractérisé en ce que** l'oxyde de chrome présente un volume poreux supérieur à 0,18 ml/g.

4. Procédé selon l'une des revendications 1 à 3 **caractérisé en ce que** l'oxyde de chrome utilisé provient de la calcination d'un précipité d'hydroxyde de chrome III ou de la réduction de l'oxyde de chrome VI.

5. Procédé selon l'une des revendications 1 à 4 dans lequel le dérivé du nickel est un oxyde, hydroxyde, halogénure, oxyhalogénure, nitrate ou sulfate de nickel Il, de préférence le chlorure de nickel.

6. Procédé selon l'une des revendications 1 à 5 dans lequel on utilise une solution aqueuse ou alcoolique d'un dérivé du nickel lors de la préparation du catalyseur.

7. Procédé selon la revendication 1 à 6 dans lequel, avant son utilisation, le catalyseur est séché sous gaz inerte ou sous air à une température comprise entre 100 et 350°C, puis activé par HF.

8. Procédé selon la revendication 7 dans lequel l'HF est d'abord introduit dilué dans de l'air ou, de préférence, dans un gaz inerte à une température allant de 150 à 200°C, puis pur à une température inférieure à 400°C, de préférence comprise entre 350 et 380°C.

9. Procédé selon d'une des revendications 1 à 8 dans lequel la température de fluoration du perchloroéthylène est comprise entre 50 et 500°C, de préférence entre 100 et 450°C et, plus particulièrement, entre 120 et 400°C

10. Procédé selon l'une des revendications 1 à 9 dans lequel le temps de contact est compris entre 3 et 100 secondes, de préférence inférieur à 30 secondes.

11. Procédé selon l'une des revendications 1 à 10 dans lequel le rapport molaire: HF/perchloroéthylène est compris entre 1/1 et 30/1, de préférence inférieur à 20/1.

12. Procédé selon l'une des revendications 1 à 11 dans lequel on opère à une pression absolue comprise entre 0,08 et 2 MPa, de préférence entre 0,1 et 1,5 MPa.

13. Procédé selon l'une des revendications 1 à 12 dans lequel on opère en présence d'un oxydant, de préférence l'oxygène ou l'air.

14. Procédé selon l'une des revendications 1 à 13 dans lequel le catalyseur, désactivé par cokage, est régénéré par traitement à l'air, à l'oxygène ou par un mélange Cl₂/HF, à une température comprise entre 250 et 400°C.

## Claims

1. Process for the catalytic fluorination of perchloroethylene by HF in the gas phase, comprising: the preparation of a bulk catalyst based on chromium and on nickel which comprises impregnation of an amorphous chromium III oxide with a solution of a nickel derivative, **characterized in that** the chromium oxide used exhibits a BET specific surface of greater than 150 m²/g and a pore volume, defined as the volume of the pores with a radius of less than 7.5 µm, of greater than 0.15 ml/g, and **in that** the Ni/Cr atomic ratio is between 0.02 and 0.4; and the use of this catalyst for the said fluorination.

2. Process according to Claim 1, **characterized in that** the chromium oxide has a BET specific surface of greater than 180 m²/g.

3. Process according to Claim 1 or 2, **characterized in that** the chromium oxide exhibits a pore volume of greater than 0.18 ml/g.

4. Process according to one of Claims 1 to 3, **characterized in that** the chromium oxide used originates from the calcination of a chromium III hydroxide precipitate or from the reduction of chromium VI oxide.

5. Process according to one of Claims 1 to 4, in which the nickel derivative is a nickel II oxide, hydroxide, halide, oxyhalide, nitrate or sulphate, preferably nickel chloride.

6. Process according to one of Claims 1 to 5, in which use is made of an aqueous or alcoholic solution of a nickel derivative during the preparation of the catalyst.

7. Process according to one of Claims 1 to 6, in which, before it is used, the catalyst is dried under an inert gas or under air at a temperature of between 100 and 350°C and then activated with HF.

8. Process according to Claim 7, in which HF is first introduced diluted in air or, preferably, in an inert gas at a temperature ranging from 150 to 200°C and then pure at a temperature of less than 400°C, preferably of between 350 and 380°C.

9. Process according to one of Claims 1 to 8, in which the temperature for the fluorination of the perchloroethylene is between 50 and 500°C, preferably between 100 and 450°C and more particularly between 120 and 400°C.

10. Process according to one of Claims 1 to 9, in which the contact time is between 3 and 100 seconds, preferably less than 30 seconds.

11. Process according to one of Claims 1 to 10, in which the molar ratio: HF/perchloroethylene is between 1/1 and 30/1, preferably less than 20/1.

12. Process according to one of Claims 1 to 11, in which the fluorination is carried out at an absolute pressure of between 0.08 and 2 MPa, preferably between 0.1 and 1.5 MPa.

13. Process according to one of Claims 1 to 12, in which the fluorination is carried out in the presence of an oxidizing agent, preferably oxygen or air.

14. Process according to one of Claims 1 to 13, in which the catalyst, deactivated by coking, is regenerated by treatment with air or with oxygen or by a Cl₂/HF mixture, at a temperature of between 250 and 400°C.

## Patentansprüche

1. Verfahren zur katalytischen Fluorierung von Perchlorethylen mit HF in der Gasphase, bei dem man: einen Vollkatalysator auf Basis von Chrom und Nickel herstellt, indem man ein amorphes Chrom-III-oxid mit einer Lösung eines Nickelderivats imprägniert, **dadurch gekennzeichnet, dass** das verwendete Chromoxid eine BET-Oberfläche von mehr als 150 m²/g und ein Porenvolumen, das als das Volumen der Poren mit einem Radius von weniger als 7,5 µm definiert ist, von mehr als 0,15 ml/g aufweist und das Ni/Cr-Atomverhältnis zwischen 0,02 und 0,4 liegt; und diesen Katalysator für die Fluorierung verwendet.

2. Verfahren nach Anspruch 1, **dadurch gekennzeichnet, dass** das Chromoxid eine BET-Oberfläche von mehr als 180 m²/g aufweist.

3. Verfahren nach Anspruch 1 oder 2, **dadurch gekennzeichnet, dass** das Chromoxid ein Porenvolumen von mehr als 0,18 ml/g aufweist.

4. Verfahren nach einem der Ansprüche 1 bis 3, **dadurch gekennzeichnet, dass** das verwendete Chromoxid aus der Calcinierung eines Chrom-IIIhydroxid-Niederschlags oder aus der Reduktion von Chrom-VI-oxid stammt.

5. Verfahren nach einem der Ansprüche 1 bis 4, bei dem es sich bei dem Nickelderivat um ein Oxid, Hydroxid, Halogenid, Oxidhalogenid, Nitrat oder Sulfat von Nickel-II, vorzugsweise Nickelchlorid, handelt.

6. Verfahren nach einem der Ansprüche 1 bis 5, bei dem man bei der Herstellung des Katalysators eine wässrige oder alkoholische Lösung eines Nickelderivats verwendet.

7. Verfahren nach einem der Ansprüche 1 bis 6, bei dem man den Katalysator vor seiner Verwendung unter Inertgas oder unter Luft bei einer Temperatur zwischen 100 und 350°C trocknet und dann mit HF aktiviert.

8. Verfahren nach Anspruch 7, bei dem man das HF zunächst in Luft oder vorzugsweise in einem Inertgas verdünnt bei einer Temperatur von 150 bis 200°C und dann rein bei einer Temperatur von weniger als 400°C, vorzugsweise zwischen 350 und 380°C, einträgt.

9. Verfahren nach einem der Ansprüche 1 bis 8, bei dem die Temperatur der Fluorierung des Perchlorethylens zwischen 50 und 500°C, vorzugsweise zwischen 100 und 450°C und spezieller zwischen 120 und 400°C liegt.

10. Verfahren nach einem der Ansprüche 1 bis 9, bei dem die Kontaktzeit zwischen 3 und 100 Sekunden und vorzugsweise unter 30 Sekunden liegt.

11. Verfahren nach einem der Ansprüche 1 bis 10, bei dem das HF/Perchlorethylen-Molverhältnis zwischen 1/1 und 30/1 und vorzugsweise unter 20/1 liegt.

12. Verfahren nach einem der Ansprüche 1 bis 11, bei dem man bei einem Absolutdruck zwischen 0,08 und 2 MPa, vorzugsweise zwischen 0,1 und 1,5 MPa, arbeitet.

13. Verfahren nach einem der Ansprüche 1 bis 12, bei dem man in Gegenwart eines Oxidationsmittels, vorzugsweise Sauerstoff oder Luft, arbeitet.

14. Verfahren nach einem der Ansprüche 1 bis 13, bei dem man den durch Koksbelegung desaktivierten Katalysator durch Behandlung mit Luft, Sauerstoff oder einer Cl₂/HF-Mischung bei einer Temperatur zwischen 250 und 400°C regeneriert.
